(19) Europäisches Patentamt
European Patent Office
Office européen des brevets

(11) Publication number: **0 295 071 A2**

(12) # EUROPEAN PATENT APPLICATION

(21) Application number: **88305215.1**

(22) Date of filing: **08.06.88**

(51) Int. Cl.4: **A 61 K 7/32**
A 61 K 7/36, A 61 K 7/38

(30) Priority: **11.06.87 US 61241**

(43) Date of publication of application:
**14.12.88 Bulletin 88/50**

(84) Designated Contracting States:
**AT BE CH DE FR GB IT LI LU NL**

(71) Applicant: **THE PROCTER & GAMBLE COMPANY**
**One Procter & Gamble Plaza**
**Cincinnati Ohio 45202 (US)**

(72) Inventor: **Tanner, Paul Robert**
**459 Dewdrop Circle, Apt. C.**
**Cincinnati Ohio 45240 (US)**

**Luebbe, John Paul**
**R.R. 2, Meadowridge Drive**
**Lawrenceburg IN 47025 (US)**

**Farris, Richard Duffy**
**5732 Mc Carthy Court**
**West Chester Ohio 45069 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

(54) **Low residue wax emulsion antiperspirant sticks.**

(57) Low residue wax emulsion antiperspirant stick compositions comprising a polar phase which is a polyhydric alcohol solubilized antiperspirant active, an emollient-containing non-polar phase which itself comprises a wax type solidifying agent and a non-polar emollient, and an emulsifier. These antiperspirant compositions leave very little visible residue on the skin and clothes, both initially upon application and later. They also have good cosmetic aesthetics and efficacy.

EP 0 295 071 A2

Bundesdruckerei Berlin

## Description

# LOW RESIDUE WAX EMULSION ANTIPERSPIRANT STICKS

## BACKGROUND OF THE INVENTION

The present invention relates to low residue wax emulsion antiperspirant stick compositions comprising a polar phase which is a polyhydric alcohol solubilized antiperspirant active, an emollient-containing non-polar phase which itself comprises a wax type solidifying agent and a non-polar emollient, and an emulsifier. Unlike conventional suspension sticks and aqueous sticks, these antiperspirant sticks leave very little visible residue on the skin and clothes, both initially upon application and later. These low residue sticks further do not produce the unpleasant cool feeling characteristic of antiperspirant products having high concentrations of water and/or ethanol. In addition, these antiperspirant sticks permit inclusion of antiperspirant actives with improved molecular distribution to provide improved antiperspirant efficacy. The present invention further relates to methods for treating or preventing perspiration and malodor associated with human underarm perspiration.

U.S. Patent 4,137,306, to Rubino et al., issued January 30, 1979, summarizes the various attempts which have been made to develop efficacious and cosmetically pleasing antiperspirant stick compositions. One such attempt has been U.S. Patent 2,890,987, to Hilfer, issued June 16, 1959, which utilized a solid fatty acid amide of an alkylolamine (e.g., a stearic acid monoethanolimide), an astringent aluminum compound, an alkylene polyhydric alcohol (e.g., propylene glycol), and relatively high levels of water. The Rubino U.S. Patent 4,137,306 sought to improve upon this formulation by removing the water and utilizing instead a low boiling polar organic solvent such as ethanol and a propoxylated alcohol as a polar emollient.

While antiperspirant sticks are old, there continues to be a need for antiperspirant stick compositions which have good cos metic aesthetics and good antiperspirant efficacy. It is therefore an object of the present invention to provide antiperspirant sticks which have excellent cosmetic properties and are easy to manufacture. A further object of the present invention is to provide antiperspirant sticks which leave very little visible residue on the skin and clothes. Another object of the present invention is to provide antiperspirant stick compositions with improved antiperspirant efficacy. An object of the present invention is also to provide methods for treating or preventing perspiration and malodor associated with human underarm perspiration.

These and other objects of the present invention will become readily apparent from the detailed description which follows.

All percentages and ratios used herein are by weight unless otherwise specified.

## SUMMARY OF THE INVENTION

The present invention relates to low residue wax emulsion antiperspirant stick compositions. These antiperspirant sticks comprise:

a) from about 20% to about 65% of a polar phase which is a polyhydric alcohol solubilized antiperspirant active, wherein said polar phase comprises:

(i) from about 10% to about 40%, by weight of the antiperspirant stick composition, of at least one polyhydric alcohol;

(ii) from about 1% to about 40%, by weight of the antiperspirant stick composition, of at least one antiperspirant active soluble in the polyhydric alcohol present in the stick composition; and

(iii) optionally from about 0% to about 20% of water, wherein the ratio of polyhydric alcohol to water is greater than about 1:1; and

(b) from about 35% to about 80% of an emollient-containing non-polar phase, wherein said non-polar phase comprises:

(i) from about 5% to about 35%, by weight of the antiperspirant stick composition, of at least one wax type solidifying agent; and

(ii) from about 10% to about 50%, by weight of the antiperspirant stick composition, of at least one non-polar emollient; and

(c) from about 0.01% to about 15% of at least one emulsifier.

The present invention further relates to methods for treating or preventing perspiration and malodor associated with underarm perspiration. These methods comprise applying to the skin of a human a safe and effective amount of a low residue wax emulsion antiperspirant stick composition of the present invention.

## DETAILED DESCRIPTION OF THE INVENTION

Low Residue Wax Emulsion Antiperspirant Stick Compositions

The low residue wax emulsion antiperspirant stick compositions of the present invention comprise the following components: (a) a polar phase which is a polyhydric alcohol solubilized antiperspirant active; (b) an emollient-containing non-polar phase which itself comprises a wax type solidifying agent and a non-polar emollient; and (c) an emulsifier. The specific components to be included in the cosmetic sticks of the present invention, and their levels, are selected in order to produce a stick of desired hardness so as to maintain dimensional stability while depositing a suitable amount of the antiperspirant active onto the skin during normal use. These components, and the weight percentages for these components, are described in detail hereinafter.

(a) Polar Phase Containing Polyhydric Alcohol Solubilized Antiperspirant Active

The compositions of the present invention essentially comprise a polar phase which is a polyhydric alcohol solubilized antiperspirant active. The polar phase of the present invention typically comprises from about 20% to about 65%, preferably from about 25% to about 60%, and more preferably from about 35% to abut 55% of the compositions herein. The polar phase comprises a polyhydric alcohol, an antiperspirant active soluble in polyhydric alcohols, and any amount of water present in the composition. Polyhydric alcohols and antiperspirant actives soluble in polyhydric alcohols, as well as optional amounts of water, are described in detail hereinafter.

(i) Polyhydric Alcohols

The polyhydric alcohols to be utilized in the present invention are one or more polyhydric alcohols selected such that the polyhyric alcohol solubilized antiperspirant active is a liquid at room temperature. Typical liquid polyhydric alcohols for use in the compositions of the present invention include: 1,2-propylene glycol; 1,3-propylene glycol; 1,3-butylene glycol (1,3-butanediol); glycerine (1,2,3-trihydroxy propane); 2-methyl-2,4-pentane-diol; and 2-ethyl-1,3-hexane diol. Preferred for use herein is glycerine, propylene glycol, and mixtures thereof. Most preferred is 1,2-propylene glycol.

The polyhydric alcohols of the present invention comprise, in total, from about 10% to about 40% of the compositions herein, preferably from about 10% to about 30%, and more preferably from about 15% to about 25%.

(ii) Antiperspirant Actives Soluble in Polyhydric Alcohols

The antiperspirant actives useful in the compositions of the present invention are antiperspirant actives soluble in the polyhydric alcohol present in the stick composition. Ther terms "soluble" and "solubilized", as used herein, mean that the antiperspirant active is dissolved in and/or colloidally dispersed (sub-micron particle size) in the polyhydric alcohol and water (if any) utilized in the composition being prepared to give a transparent or semitransparent liquid. Typically the transparency of the liquid is such that more than 50%, preferably more than 75%, of 500 nm light is transmitted through the liquid as measured by a standard UV-visible absorption instrument (relative to the polyhydric alcohol and water (if any) without the antiperspirant active). Antiperspirant actives soluble in alcohols are known, having been disclosed, for example, in U.S. Patent 4,137,306, to Rubino et al, issued January 30, 1979, the disclosures of which are incorporated herein by reference in their entirety.

Preferred antiperspirant actives useful herein include the following. Polyhydroxy complexes of basic aluminum salts as described in U.S. Patent 3,420,932 to Jones et al, issued January 7, 1969; U.S. Patent 3,359,169, to Slater et al, issued December 19, 1967; U.S. Patent 3,523,130, to Jones et al, issued August 4, 1970; U.S. Patent 3,507,896, to Jones et al, issued April 21, 1970; U.S. Patent 3,873,686, to Beekman, issued March 25, 1975; U.S. Patent 3,876,758, to Beekman, issued April 8, 1975; and Britain Patent Specification 1,159,685, to Armour Pharmaceutical Co., published July 30, 1969 (all these disclosures being incorporated herein by reference in their entirety) and commercially-available as Rehydrol and Rehydrol II (supplied by Reheis Chemical Co.). Polyhydroxy derivatives of zinc and zirconium complexes of basic aluminum halides as described in U.S. Patent 3,405,153, to Jones et al, issued October 8, 1968; U.S. Patent 3,555,146, to Jones et al, issued January 12, 1971; Britain Patent Specification 1,159,686, to Jones et al, published July 30, 1969 (all these disclosures being incorporated herein by reference in their entirety). Zirconyl hydroxychloride salts, especially zirconium-aluminum-glycine complexes ("ZAG complexes"), as described in the following patent documents, all incorporated by reference herein in their entirety: Belgium Patent Specification 825,146, to Schmitz, issued August 4, 1975; U.S. Patent 2,814,585, to Daley, issued November 26, 1957; U.S. Patent 3,679,068, to Luedders et al, issued February 12, 1974; U.S. Patent 4,017,599, to Rubino, issued April 12, 1977; U.S. Patent 4,120,948, to Shelton, issued October 17, 1978; and Britain Patent Specification 2,144,992, to Callaghan et al, published March 20, 1985. Aluminum chlorhydroxide ("ACH") salts as described in the following documents, all incorporated by reference herein in their entirety: U.S. Patent 3,887,692, to Gilman, issued June 3, 1975; U.S. Patent 3,904,741, to Jones et al, issued September 9, 1975; U.S. Patent 4,359,456, to Gosling et al, issued November 16, 1982; Britain Patent Specification 2,048,229, to Fitzgerald et al, published December 10, 1980; and Britain Patent Specification 1,347,950, to Shin et al, published February 27, 1974.

Particularly preferred antiperspirant actives useful herein are those with enhanced efficacy due to improved molecular distributions, especially polyhydric alcohol solubilized ACH salts, polyhydric alcohol solubilized zirconyl hydroxychloride salts, and mixtures thereof (especially ZAG complexes), as described in copending

U.S. Patent Application Serial No. (P&G Case 3672), by Tanner et al., "Liquid Antiperspirant Actives and Processes for Preparing the Same", filed June 11, 1987 (incorporated by reference herein in its entirety). Aluminum chlorhydroxide salts, zirconyl hydroxychloride salts, and mixtures thereof having improved molecular distributions are known, having been disclosed, for example, in the following documents, all incorporated by reference herein in their entirety: U.S. Patent 4,359,456, to Gosling et al, issued November 16, 1982; European Patent Application Publication No. 6,739, to Unilever Limited, published January 9, 1980; European Patent Application Publication No. 183,171, to Armour Pharmaceutical Company, published June 4, 1986; British Patent Specification 2,048,229, to The Gillette Company, published December 10, 1980; European Patent Application Publication No. 191,628, to Unilever PLC, published August 20, 1986; and British Patent Specification 2,144,992 to The Gillette Company, published March 20, 1985. Antiperspirant actives with enhanced efficacy due to improved molecular distribution are also described in European Patent Application Publication No. 7,191, to Unilever Limited, published January 23, 1980, incorporated by reference herein in its entirety.

The improved molecular distribution is determined by the known analysis method called gel permeation chromatography. This analysis method is described, for example, in several of the above-incorporated patent specifications. It is preferred for purposes of the present invention that the antiperspirant active soluble in the polyhydric alcohol have enhanced efficacy due to improved molecular distribution having the ratio of peak 3 to peak 2 greater than about 0.1:1 as determined by gel permeation chromatography. This ratio, as is recognized by one skilled in the art, relates to the relative area under these two peaks as measured by the gel permeation chromatography analysis method.

The antiperspirant active soluble in polyhydric alcohols typically comprise in total from about 1% to about 40%, preferably from about 10% to about 30%, and most preferably from about 10% to about 25%, of the compositions of the present invention.

(iii) Water

An optional component of the compositions of the present invention is water. Water may comprise from about 0% to about 20%, preferably from about 0% to about 15%, and more preferably from about 5% to about 15%, of the compositions herein. Furthermore, for purposes of the present invention the ratio of polyhydric alcohol to water is preferably greater than about 1:1, and more preferably greater than about 2:1.

The amount of water present in the compositions herein is that amount which is detected by Karl-Fisher moisture analysis. Thus, the water content of the compositions of the present invention includes all of the free water added to the composition during formulation (by "free water" is meant water not chemically bound to the antiperspirant active being used to prepare the composition) and any amount of water being used to the active during formulation which is detectable by Karl-Fisher moisture analysis and any other water present in the composition which is detectable by Karl-Fisher moisture analysis.

The compositions of the present invention typically comprise some amount of water. However, the compositions herein also include those compositions to which no free water has been added, and those compositions in which there is no water present at all (either free water or water bound to the antiperspirant active).

(b) Emollient-Containing Non-Polar Phase:

The compositions of the present invention also essentially comprise an emollient-containing non-polar phase. The non-polar phase of the present invention typically comprises from about 35% to about 80%, preferably from about 40% to about 75%, and more preferably from about 45% to about 65% of the compositions herein. This phase comprises a wax type solidifying agent and a non-polar emollient as described hereinafter.

(i) Wax Type Solidifying Agent

An essential component of the compositions herein is at least one wax type solidifying agent. Among such wax type solidifying agents useful herein are the high melting point waxes having a melting point of from about 65° C to about 102° C, low melting point waxes having a melting point of from about 37° C to about 75° C, and preferably mixtures thereof.

High melting point waxes include beeswax, spermaceti, carnauba, baysberry, candelilla, montan, ozokerite, ceresin, paraffin, hydrogenated castor oil (castor wax), synthetic waxes such as Fisher-Tropsch waxes, microcrystaline waxes, and mixtures thereof. Castor wax is a preferred high-melting point wax useful herein. Such high-melting point waxes among those useful herein are disclosed in U.S. Patent 4,049,792, to Elsnau, issued September 20, 1977 (incorporated by reference herein).

Low melting point waxes include fatty acids, fatty alcohols, fatty acid esters and fatty acid amides, having fatty chains of from about 8 to about 30 carbon atoms, preferably from about 12 to about 18 carbon atoms, and mixtures thereof. Preferred low melting point waxes include cetyl alcohol, palmitic acid, myristyl alcohol, stearyl alcohol, paraffin, cetyl stearate, cetyl palmitate, cetyl myristate, stearyl stearate, and mixtures thereof. Cetyl stearate, stearyl alcohol, cetyl alcohol, cetyl palmitate, cetyl myristate, stearyl stearate and mixtures thereof are particularly preferred.

Wax type solidifying agents among those useful in the sticks of this invention are disclosed in the following patent specifi cations, all of which are incorporated by reference herein in their entirety: U.S. Patent 4,049,792,

to Elsnau, issued September 20, 1977; U.S. Patent 4,151,272, to Geary et al, issued April 24, 1975; U.S. Patent 4,229,432, to Geria, issued October 21, 1980; U.S. Patent 4,280,994, to Turney, issued July 28, 1981; U.S. Patent 4,126,679, to Davy et al, issued November 21, 1978; and European Patent Application Publication Number 117,070, to May, published August 29, 1984.

The wax type solidifying agents typically comprise in total from about 5% to about 35%, preferably from about 10% to about 30%, and more preferably from about 15% to about 30% by weight of the compositions of the present invention.

(ii) Non-Polar Emollients

The compositions of the present invention further essentially comprise at least one non-polar emollient. Preferred non-polar emollients are volatile silicone oils, non-polar non-volatile emollients, and mixtures thereof. The compositions of the present invention more preferably comprise at least one volatile silicone oil which functions as a liquid emollient, or especially a mixture of volatile silicone oils and non-polar non-volatile emollients. The term "volatile", as used herein, refers to those materials which have a measurable vapor pressure at ambient temperature.

Volatile silicone oils useful in the cosmetic stick compositions of the present invention are preferably cyclic or linear polydimethylsiloxanes containing from about 3 to about 9, preferably from about 4 to about 5, silicon atoms. The following formula illustrates cyclic volatile polydimethylsiloxanes useful in the cosmetic stick compositions disclosed herein:

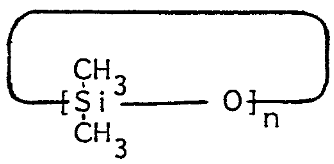

wherein n equals about 3 to about 7. Linear polydimethylsiloxanes contain from about 3 to about 9 silicon atoms per molecule and have the following general formula:

$(CH_3)_3Si-O-[Si(CH_3)_2-O]_n-Si(CH_3)_3$

wherein n equals about 1 to about 7. Linear volatile silicone materials generally have viscosities of less than about 5 centistrokes at 25°C while cyclic materials typically have viscosities of less than about 10 centistokes. A description of various volatile silicone oils is found in Todd, et al., "Volatile Silicone Fluids for Cosmetics", Cosmetics & Toiletries, 91, pages 27-32 (1976), the disclosures of which are incorporated by reference herein in their entirety.

Examples of preferred volatile silicone oils useful herein include: Dow Corning 344, Dow Corning 345, and Dow Corning 200 (manufactured by the Dow Corning Corp.); Silicone 7207 and Silicone 7158 (manufactured by the Union Carbide Corp.); SF 1202 (manufactured by General Electric); and SWS-03314 (manufactured by SWS Silicones, Inc.).

The present antiperspirant compositions in stick form also preferably contain one or more non-polar non-volatile emollients. Such materials include fatty acid and fatty alcohol esters, hydrocarbons, non-volatile silicone oils, and mixtures thereof. Emollients among those useful herein are described in 1 Cosmetics, Science and Technology 27-104 (M. Balsam and E. Sagarin, Ed.; 1972), and U.S. Patent 4,202,879, to Shelton, issued May 13, 1980 (both incorporated by reference herein).

Non-volatile silicone oils useful as an emollient material include polyalkylsiloxanes, polyalkylarylsiloxanes, and polyethersiloxane copolymers. The essentially non-volatile polyalkyl siloxanes useful herein include, for example, polydimethyl siloxanes with viscosities of from about 5 to about 100,000 centitokes at 25°C. Among the preferred non-volatile emollients useful in the present compositions are the polydimethyl siloxanes having viscosities from about 10 to about 400 centistokes at 25°C. Such polyalkyl siloxanes include the Vicasil series (sold by General Electric Company) and the Dow Corning 200 series (sold by Dow Corning Corporation). Polyalkylaryl siloxanes include poly methyl phenyl siloxanes having viscosities of from about 15 to about 65 centistokes at 25°C. These are available, for example, as SF 1075 methylphenyl fluid (sold by General Electric Company) and 556 Cosmetic Grade Fluid (sold by Dow Corning Corporation). Useful polyether siloxane copolymers include, for example, a polyoxyalkylene ether copolymer having a viscosity of about 1200 to 1500 centistokes at 25°C. Such a fluid is available as SF-1066 organosilicone surfactant (sold by General Electric Company). Polysiloxane ethylene glycol ether copolymers are preferred copolymers for use in the present compositions.

Non-polar fatty acid and fatty alcohol esters useful herein as an emollient material include, for example, di-isopropyl adipate, isopropyl myristate, isopropyl palmitate, and PPG-2 myristyl ether propionate. Hydrocarbons such as isohexadecane (e.g., Permethyl 101A supplied by Presperse) are also useful as non-polar emollients.

The emollients typically comprise in total from about 10% to about 50%, preferably from about 15% to about 45%, and more preferably from about 20% to about 40% by weight of the compositions of the present invention.

(c) Emulsifier

The compositions of the present invention also essentially comprise at least one emulsifier. Preferred is the use of a dimethicone copolyol, an organic emulsifier having an HLB value within the range of from about 1 to about 10, or a mixture of a dimethicone copolyol and an organic emulsifier.

The most preferred emulsifier for use in the compositions of the present invention is at least one silicone-containing material referred to herein as "dimethicone copolyol" which is one or more polyalkylene oxide modified dimethylpolysiloxanes. The dimethicone copolyols include the polyalkylene oxide modified dimethylpolysiloxanes of the following formulae:

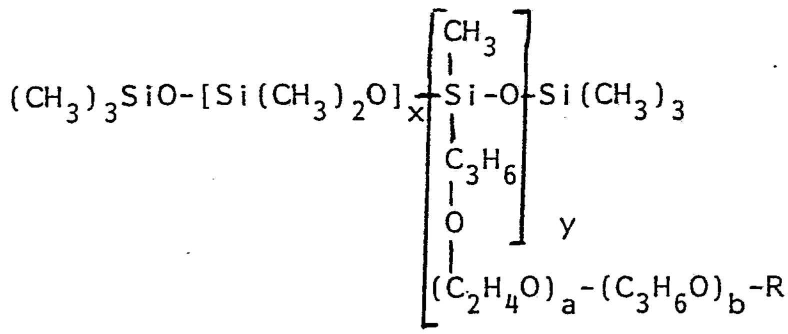

and

$$R'-Si-[[OSi(CH_3)_2]_x-(OC_2H_4)_a-(OC_3H_6)_b-OR'']_3$$

wherein R is hydrogen, an alkyl group having from about 1 to about 12 carbon atoms, an alkoxy group having from about 1 to about 6 carbon atoms, or a hydroxyl group; R′ and R″ are alkyl groups having from 1 to about 12 carbon atoms; x is an integer of from 1 to 100, preferably from 20 to 30; y is an integer of 1 to 20, preferably from 2 to 10; and a and b are integers of from 0 to 50, preferably from 20 to 30.

Dimethicone copolyols among those useful herein are disclosed in the following patent documents, all incorporated by reference herein in their entirety: U.S. Patent 4,122,029, to Gee et al., issued October 24, 1978; U.S. Patent 4,265,878, to Keil, issued May 5, 1981; and U.S. Patent 4,421,769, to Dixon et al., issued December 20, 1983. Commercially available dimethicone copolyols, useful herein, include Silwet Surface Active Copolymers (manufactured by the Union Carbide Corp.), Dow Corning Silicone Surfactants (manufactured by the Dow Corning Corp.); Silicone Copolymer F-754 (manufactured by SWS Silicones Corp.); and Rhodorsil 70646 Fluid (manufactured by Rohne Poulenc, Inc.). Dow Corning Q2-3225C Silicone Fluid is a preferred dimethicone copolyol.

Also preferred for use herein are organic emulsifiers, either alone or in combination with a dimethicone copolyol. If utilized alone, the organic emulsifier preferably has an HLB value within the range of from about 1 to about 10. The HLB (short for "Hydrophile-Lipophile Balance") value system is fully described, and values for various materials are provided, in the publication The HLB System, A Time-Saving Guide to Emulsifier Selection (published by ICI Americas Inc., Wilmington, Delaware; 1984), the disclosures of which are incorporated herein by reference in their entirety. Examples of useful organic emulsifiers are sorbitan esters.

The emulsifier typically comprises in total from about 0.01% to about 15%, preferably from about 0.05% to about 10%, and most preferably from about 0.05% to about 5%, of the compositions of the present invention.

Optional Components

The compositions of the present invention may also contain optional components which modify the physical characteristics of the composition, or serve as "active" components when deposited on the skin in addition to the antiperspirant active. Additional active components include bacteriostats and fungistats. The particular non-active components that may be useful will typically depend upon the cosmetic properties that are desired. Such components include, for example, colorants and perfumes. Another optional component is a polar emollient such as, for example, propylene carbonate. While the compositions herein may also contain as an optional component some amount of a low boiling monohydric alcohol (e.g., ethanol; isopropanol), for reasons of cosmetic aesthetics it is preferred the compositions of the present invention contain less than about 10%, more preferably less than about 5%, and most preferably about 0%, of a low boiling monohydric alcohol. Optional components useful herein are described in the following documents, all incorporated by reference herein: U.S. Patent 4,049,792, to Elsnau, issued September 20, 1977; Canadian Patent 1,164,347, to Beckmeyer, et al., issued March 27, 1984; European Patent Specification 117,070, to May, published August 29, 1984; and Geria, "Formulation of Stick Antiperspirants and Deodorants," 99 Cosmetics & Toiletries 55-60 (1984).

The specific essential and optional materials to be included in specific stick compositions of the present

invention, and their levels, are selected in order to produce a stick of desired hardness so as to maintain dimensional stability while depositing a suitable amount of active material on the skin during normal use. Hardness of sticks can be determined in a variety of methods, including American Society for Testing Materials (ASTM) Method D-5. This method involves the use of a needle or polished cone of particular weight and dimension, which is allowed to travel downward through the stick material for a predetermined period of time. The distance traveled by the needle or cone is a relative measure of the stick hardness. Utilizing Method D-5, with a penetration cone (Model H1310; sold by Humboldt Manufacturing Company) weighing 2.52 grams, and a Precision Model 14AN-8 Penetrometer (sold by GCA Corp.), the cosmetic sticks of the present invention preferably yield a penetration value of from about 3.0 to about 20.0 millimeters, more preferably from about 5.0 to about 15.0 millimeters, over a period of 5 seconds. These values represent an average penetration for sticks within a given production batch, since such penetration values may vary from stick to stick within the batch.

Another measure of the hardness of the preferred cosmetic sticks of the present invention is a break strength measurement. The break strength is determined using a Velmex Inc. Model B2509BJ (sold by Crown Tool & Supply Co.). In this instrument the force gauge is attached to a slide which allows the gauge to contact the test stick through a breaker bar at a speed of 3.3 inches per minute. The value recorded is the force gauge reading when the stick breaks. The cosmetic sticks of the present invention preferably have break strengths within the range of from about 3 to about 15 pounds.

Methods for Preventing Perspiration and Malodor

The present invention also provides methods for treating or preventing perspiration and malodor associated with human underarm perspiration. These methods comprise applying to the skin of a human a safe and effective amount of a low residue wax emulsion antiperspirant stick composition of the present invention. The term "a safe and effective amount", as used herein, is an amount which is effective in eliminating or substantially reducing perspiration and malodor associated with human underarm perspiration while being safe for human use at a reasonable risk/benefit ratio.

The following examples further describe and demonstrate embodiments within the scope of the present invention. The examples are given solely for the purpose of illustration and are not to be construed as limitations of the present invention as many variations thereof are possible without departing from the spirit and scope.

## EXAMPLE I

An antiperspirant stick composition of the present invention is prepared comprising the following ingredients:

| Components | Weight % |
| --- | --- |
| Cyclomethicone D-5[1)] | 26.0 |
| Isohexadecane[2)] | 5.0 |
| Dow Corning Q2-3225C[3)] | 1.0 |
| Cetyl Stearate[4)] | 22.0 |
| Castor Wax MP70[5)] | 1.0 |
| Propylene Glycol[6)] | 18.0 |
| Aluminum Zirconium Trichlorohydrex Glycine[7)] | 22.5 |
| Water | 4.5 |

1) Supplied by GE Silicones

2) Permethyl 101A supplied by Presperse and made by Permethyl Corp.

3) A mixture of cyclomethicone and dimethicone copolyol (ratio of approximately 9:1) supplied by Dow Corning

4) Schercemol CS supplied by Scher Chemicals

5) Hydrogenated castor oil supplied by CAS Chemicals

6) Propylene glycol (U.S.P.) supplied by Union Carbide

7) Improved distribution ZAG supplied by Westwood (approximately 20% water)

This stick composition is prepared as follows. All the ingredients except the solubilized active (i.e., the propylene glycol, ZAG, and water) are combined and heated to 180°F (82°C). The solubilized active is then prepared by milling the ZAG into a 122°F (50°C) mixture of the water and propylene glycol until all the ZAG powder is dissolved. The oil and wax mixture is then cooled to 117°F (47°C) and the solubilized active is slowly added with milling. This composition is cooled with agitation to 113°F (45°C) and poured into canisters. A uniform antiperspirant stick forms as the composition cools to room temperature.

This antiperspirant composition is applied to the underarm skin of a human to effectively prevent perspiration and underarm odor resulting from perspiration. The composition is relatively non-sticky and glides on the skin smoothly. It also produces relatively little visible white residue on the skin and clothes, both initially upon application and later.

## EXAMPLE II

An antiperspirant stick composition of the present invention is prepared comprising the following ingredients.

| Components | Weight % |
|---|---|
| Cyclomethicone D-5[1)] | 14.0 |
| Isohexadecane[2)] | 10.0 |
| Dow Corning Q2-3225C[3)] | 2.0 |
| Cetyl Stearate[4)] | 25.0 |
| Castor Wax MP70[5)] | 3.0 |
| Castor Wax MP80[5)] | 1.0 |
| Propylene Glycol[6)] | 20.0 |
| Aluminum Zirconium Tetrachlorohydrex Glycine[7)] | 20.0 |
| Water | 5.0 |

1) Supplied by GE Silicones
2) Permethyl 101A supplied by Presperse and made by Permethyl Corp.
3) A mixture of cyclomethicone and dimethicone copolyol (ratio of approximately 9:1) supplied by Dow Corning
4) Schercemol CS supplied by Scher Chemicals
5) Hydrogenated castor oil supplied by CAS Chemicals
6) Propylene glycol (U.S.P.) supplied by Union Carbide
7) Improved distribution ZAG supplied by Wickhen (approximately 20% water)

This stick composition is prepared by essentially the same procedure as described in Example I.

This antiperspirant composition is applied to the underarm skin of a human to effectively prevent perspiration and underarm odor resulting from perspiration. The composition is relatively non-sticky and glides on the skin smoothly. It also produces relatively little visible white residue on the skin and clothes, both initially upon application and later.

EXAMPLE III

An antiperspirant stick composition of the present invention is prepared comprising the following ingredients.

| Components | Weight % |
|---|---|
| Cyclomethicone D-5[1)] | 27 |
| Cetyl Stearate[2)] | 20 |
| Castor Wax[3)] | 3 |
| Dow Corning Q2-3225C[4)] | 5 |
| Propylene Glycol[5)] | 35 |
| Rehydrol II[6)] | 10 |

1) Supplied by GE Silicones

2) Schercemol CS supplied by Scher Chemicals

3) Hydrogenated castor oil supplied by CAS Chemicals

4) A mixture of cyclomethicone and dimethicone copolyol (ratio of approximately 9:1) supplied by Dow Corning

5) Propylene glycol (U.S.P.) supplied by Union Carbide

6) Propylene glycol complexed aluminum chlorhydrate (approximately 25% propylene glycol) supplied by Reheis Chemical Company.

This stick composition is prepared by essentially the same procedure as described in Example I.

This antiperspirant composition is applied to the underarm skin of a human to effectively prevent perspiration and underarm odor resulting from perspiration. The composition is relatively non-sticky and glides on the skin smoothly. It also produces relatively little visible white residue on the skin and clothes, both initially upon application and later.

## Claims

1. Low residue wax emulsion antiperspirant compositions in stick form comprising:

(a) from about 20% to about 65% of a polar phase which is a polyhydric alcohol solubilized antiperspirant active, wherein said polar phase comprises:

() from about 10% to about 40%, by weight of the antiperspirant stick composition, of at least one polyhydric alcohol;

(ii) from about 1% to about 40%, by weight of the antiperspirant stick composition, of at least one antiperspirant active soluble in the polyhydric alcohol present in the stick composition; and

(iii) optionally from about 0% to about 20% of water, wherein the ratio of polyhydric alcohol to water is greater than about 1:1 and

(b) from about 35% to about 80% of an emollient-containing non-polar phase, wherein said non-polar phase comprises:

(i) from about 5% to about 35%, by weight of the antiperspirant stick composition, of at least one wax type solidifying agent, and

(ii) from about 10% to about 50%, by weight of the antiperspirant stick composition, of at least one non-polar emollient; and

(c) from about 0.01% to about 15% of at least one emulsifier.

2. Low residue wax emulsion antiperspirant compositions in stick form according to Claim 1 wherein the polyhydric alcohol is selected from glycerine, propylene glycol, and mixtures thereof; and the non-polar emollient is selected from volatile silicone oils and mixture of volatile silicone oils and non-polar non-volatile emollients.

3. Low residue wax emulsion antiperspirant compositions in stick form according to Claim 2 wherein the antiperspirant active soluble in the polyhydric alcohol is selected from the group consisting of polyhydroxy complexes of basic aluminum salts, polyhydroxy derivatives of zinc and zirconium complexes

of basic aluminum halides, zirconyl hydroxychloride salts, aluminum chlorhydroxide salts, and mixtures thereof.

4. Low residue wax emulsion antiperspirant compositions in stick form according to Claim 3 wherein the antiperspirant active soluble in the polyhydric alcohol is selected from the group consisting of aluminum chlorhydroxide salts, zirconium-aluminum-glycine complexes, and mixtures thereof.

5. Low residue wax emulsion antiperspirant compositions in stick form according to Claim 4 wherein the aluminum chlorhydroxide salts and zirconium-aluminum-glycine complexes have enhanced efficacy due to improved molecular distribution.

6. Low residue wax emulsion antiperspirant compositions in stick form according to Claim 5 wherein the emulsifier is a dimethicone copolyol.

7. Low residue wax emulsion antiperspirant compositions in stick form comprising:

a) from about 25% to about 60% of a polar phase which is a polyhydric alcohol solubilized antiperspirant active, wherein said polar phase comprises:

(i) from about 10% to about 30%, by weight of the antiperspirant stick composition, of a polyhydric alcohol selected from the group consisting of glycerine, propylene glycol, and mixtures thereof;

(ii) from about 10% to about 30%, by weight of the antiperspirant stick, of at least one antiperspirant active soluble in the polyhydric alcohol present in the stick composition selected from the group consisting of polyhydroxy complexes of basic aluminum salts, polyhydroxy derivatives of zinc and zirconium complexes of basic aluminum halides, zirconyl hydroxychloride salts, aluminum chlorhydroxide salts, and mixtures thereof; and

(iii) optionally from about 0% to about 15% of water, wherein the ratio of polyhydric alcohol to water is greater than about 1:1; and

(b) from about 40% to about 75% of an emollient-containing non-polar phase, wherein said non-polar phase comprises:

(i) from about 10% to about 30%, by weight of the antiperspirant stick composition, of at least one wax type solidifying agent; and

(ii) from about 15% to about 45%, by weight of the antiperspirant stick composition, of at least one non-polar emollient; and

(c) from about 0.05% to about 10% of at least one emulsifier.

8. Low residue wax emulsion antiperspirant compositions in stick form according to Claim 7 wherein the non-polar emollient is selected from volatile silicone oils and mixtures of volatile silicone oils and non-polar non-volatile emollients.

9. Low residue wax emulsion antiperspirant compositions in stick form according to Claim 8 wherein the antiperspirant active soluble in the polyhydric alcohol is selected from zirconium-aluminum-glycine complexes, aluminum chlorhydroxide salts, and mixtures thereof.

10. Low residue wax emulsion antiperspirant compositions in stick form according to Claim 9 wherein the zirconium-aluminum-glycine complexes and aluminum chlorhydroxide salts have enhanced efficacy due to improved molecular distribution.

11. Low residue wax emulsion antiperspirant compositions in stick form according to Claim 10 wherein the emulsifier is a dimethicone copolyol.

12. Low residue wax emulsion antiperspirant compositions in stick form comprising:

(a) from about 35% to about 55% of a polar phase which is a polyhydric alcohol solubilized antiperspirant active, wherein said polar phase comprises:

(i) from about 10% to about 30%, by weight of the antiperspirant stick composition, of a polyhydric alcohol selected from propylene glycol;

(ii) from about 10% to about 30%, by weight of the antiperspirant stick composition, of at least one anti- perspirant active soluble in the propylene glycol present in the stick composition; and

(iii) optionally from about 0% to about 15% of water, wherein the ratio of propylene glycol to water is greater than about 1:1; and

(b) from about 45% to about 65% of an emollient-containing non-polar phase, wherein said non-polar phase comprises:

(i) from about 10% to about 30%, by weight of the antiperspirant stick composition, of at least one wax type solidifying agent; and

(ii) from about 15% to about 45%, by weight of the antiperspirant stick composition, of at least one non-polar emollient; and

c) from about 0.05% to about 10% of at least one emulsifier.

13. Low residue wax emulsion antiperspirant compositions in stick form according to Claim 12 wherein the antiperspirant active soluble in the propylene glycol is selected from the group consisting of polyhydroxy complexes of basic aluminum salts, polyhydroxy derivatives of zinc and zirconium complexes of basic aluminum halides, zirconyl hydroxychloride salts, aluminum chlorhydroxide salts, and mixtures thereof.

14. Low residue wax emulsion antiperspirant compositions in stick form according to Claim 13 wherein the antiperspirant active soluble in the propylene glycol is selected from zirconium-aluminum-glycine complexes, aluminum chlorhydroxide salts, and mixtures thereof.

15. Low residue wax emulsion antiperspirant compositions in stick form according to Claim 14 wherein the zirconium-aluminum-glycine complexes and the aluminum chlorhydroxide salts have enhanced efficacy due to improved molecular distribution.

16. Low residue wax emulsion antiperspirant compositions in stick form according to Claim 15 wherein the non-polar emollient is selected from volatile silicone oils and mixtures of volatile silicone oils and non-polar non-volatile emollients; and wherein further the emulsifier is a dimethicone copolyol.

17. Low residue wax emulsion antiperspirant compositions in stick form comprising:

(a) from about 35% to about 55% of a polar phase which is a polyhydric alcohol solubilized antiperspirant active, wherein said polar phase comprises:

(i) from about 15% to about 25%, by weight of the antiperspirant stick composition, of a polyhydric alcohol selected from propylene glycol;

(ii) from about 10% to about 25%, by weight of the antiperspirant stick composition, of at least one antiperspirant active soluble in the propylene glycol present in the stick composition selected from the group consisting of zirconium-aluminum-glycine complexes having enhanced efficacy due to improved molecular distribution, aluminum chlorhydroxide salts having enhanced efficacy due to improved molecular distribution, and mixtures thereof; and

(iii) from about 5% to about 15% of water, wherein the ratio of propylene glycol to water is greater than about 2:1; and

(b) from about 45% to about 65% of an emollient-containing non-polar phase, wherein said non-polar phase comprises;

(i) from about 15% to about 30%, by weight of the antiperspirant stick composition, of at least one wax type solidifying agent; and

(ii) from about 20% to about 40%, by weight of the antiperspirant stick composition, of at least one non-polar emollient selected from volatile silicone oils and mixtures of volatile silicone oils and non-polar non-volatile emollients; and

(c) from about 0.05% to about 5% of at least one emulsifier selected from dimethicone copolyol.

18. Methods for treating or preventing perspiration and malodor associated with human underarm perspiration, said methods comprising applying to the skin of a human a safe and effective amount of a low residue wax emulsion antiperspirant stick composition according to Claim 1.

19. Methods for treating or preventing perspiration and malodor associated with human underarm perspiration, said methods comprising applying to the skin of a human a safe and effective amount of a low residue wax emulsion antiperspirant stick composition according to Claim 7.

20. Methods for treating or preventing perspiration and malodor associated with human underarm perspiration, said methods comprising applying to the skin of a human a safe and effective amount of a low residue wax emulsion antiperspirant stick composition according to Claim 12.

21. Methods for treating or preventing perspiration and malodor associated with human underarm perspiration, said methods comprising applying to the skin of a human a safe and effective amount of a low residue wax emulsion antiperspirant stick composition according to Claim 17.